# EUROPEAN PATENT APPLICATION

(11) **EP 4 708 319 A2**
(43) Date of publication of application: **11.03.2026**
(21) Application number: 26153815.1
(22) Date of filing: 15.04.2022
(51) Int. Cl.: G16H 40/67

(54) **GLOBAL CONFIGURATION SERVICE**

(30) Priority: 15.04.2021 US 202163175199 P
(62) Divisional of application: 22722095.1
(71) Applicant: DexCom, Inc., San Diego, CA 92121 (US)
(72) Inventor: SANIGEPALLI, Praveen Kumar, San Diego, California, 92121 (US); ALVES, Ricardo, San Diego, California, 92121 (US); RHOUDA, El Mostafa, San Diego, California, 92121 (US); SMITH, Brian, San Diego, California, 92121 (US); SMITH, Daniel, San Diego, California, 92121 (US)
(74) Representative: Crowell & Moring U.K. LLP

(57) **Abstract**

Certain aspects of the present disclosure relate generally to configuring applications used in conjunction with medical devices in order to help with monitoring and improving the patient's health. Certain aspects include a method including receiving a request for assets including access information associated with a user of a computing device, the access information including feature customization information for a health intervention application and being issued by an account management service for the health intervention application, and validating that the access information is valid. The method may also include responsive to the validating, generating configuration information identifying a set of assets with which the health intervention application is to be provisioned based, at least in part, on the feature customization information included in the access information, and transmitting the configuration information to the health intervention application to configure the health intervention application with the identified set of assets.

## Description

### CROSS-REFERENCE TO RELATED APPLICATIONS

This application claims the benefit of U.S. Provisional Application No. 63/175,199 entitled "GLOBAL CONFIGURATION SERVICE," which was filed on April 15, 2021. The aforementioned application is herein incorporated by reference in its entirety.

### BACKGROUND

Diabetes is a metabolic condition relating to the production or use of insulin by the body. Insulin is a hormone that allows the body to use glucose for energy, or store glucose as fat.

When a person eats a meal that contains carbohydrates, the food is processed by the digestive system, which produces glucose in the person's blood. Blood glucose can be used for energy or stored as fat. The body normally maintains blood glucose levels in a range that provides sufficient energy to support bodily functions and avoids problems that can arise when glucose levels are too high, or too low. Regulation of blood glucose levels depends on the production and use of insulin, which regulates the movement of blood glucose into cells.

When the body does not produce enough insulin, or when the body is unable to effectively use insulin that is present, blood sugar levels can elevate beyond normal ranges. The state of having a higher than normal blood sugar level is called "hyperglycemia." Chronic hyperglycemia can lead to a number of health problems, such as cardiovascular disease, cataract and other eye problems, nerve damage (neuropathy), and kidney damage. Hyperglycemia can also lead to acute problems, such as diabetic ketoacidosis-a state in which the body becomes excessively acidic due to the presence of blood glucose and ketones, which are produced when the body cannot use glucose. The state of having lower than normal blood glucose levels is called "hypoglycemia." Severe hypoglycemia can lead to acute crises that can result in seizures or death.

A diabetes patient can receive insulin to manage blood glucose levels. Insulin can be received, for example, through a manual injection with a needle. Wearable insulin pumps are also available. Diet and exercise also affect blood glucose levels.

Diabetes conditions are sometimes referred to as "Type 1" and "Type 2." A Type 1 diabetes patient is typically able to use insulin when it is present, but the body is unable to produce sufficient amounts of insulin, because of a problem with the insulin-producing beta cells of the pancreas. A Type 2 diabetes patient may produce some insulin, but the patient has become "insulin resistant" due to a reduced sensitivity to insulin. The result is that even though insulin is present in the body, the insulin is not sufficiently used by the patient's body to effectively regulate blood sugar levels.

Management of diabetes can present complex challenges for patients, clinicians, and caregivers, as a confluence of many factors can impact a patient's glucose level and glucose trends. To assist patients with better managing this condition, a variety of health or disease intervention software applications (hereinafter "applications") have been developed by various providers. Diabetes is one example of a condition for which a variety of software applications have been developed. Numerous other applications have been developed for other health-related conditions. For example, these applications may include intervention applications for supporting the treatment of other diseases or applications that help with improving a patient's health overall, such as activity tracking applications, dieting applications, and the like.

These health applications are used in conjunction with various sensors measuring biological parameters, as well as tracking events such as activity and nutrition to provide the user with information regarding the user's health state as well as disease or health progress. In most cases, applications are designed specifically for each use case to achieve the specific goals relating to the use case for a specific set of users. This use-case specific approach is especially important with regard to safety critical information as different regulations or considerations must be applied to determine how the application interacts with a patient's health related information. These regulations may, for example, be dependent on geographical locations, or based on capabilities or specifications around the patient's specific demographics or health state.

This background is provided to introduce a brief context for the summary and detailed description that follow. This background is not intended to be an aid in determining the scope of the claimed subject matter nor be viewed as limiting the claimed subject matter to implementations that solve any or all of the disadvantages or problems presented above.

### SUMMARY

Certain aspects provide a method for configuring a diabetes intervention application executing on a computing device via a remote server, comprising: receiving, from the diabetes intervention application, a request for assets provided by the remote server, the request including access information associated with a user of the computing device, the access information including feature customization information for the diabetes intervention application and being issued by an account management service for the diabetes intervention application; validating that the access information is valid; responsive to validating that the access information is valid, generating configuration information identifying a set of assets with which the diabetes intervention application is to be provisioned based, at least in part, on the feature customization information included in the access information; and transmitting the configuration information to the diabetes intervention application to configure the diabetes intervention application with the identified set of assets.

In the above method, the feature customization information comprises location information associated with a geographical location of the user of the computing device. In the above method, the generating comprises: based on the feature customization information, identifying, from a plurality of uniform resource locators (URLs), a set of URLs to be used for configuring the diabetes intervention application, wherein the configuration information includes the set of URLs. In the above method, the generating comprises: based on the feature customization information, identifying, from a plurality of application features, a set of application features to be activated for configuring the diabetes intervention application, wherein the configuration information includes one or more flags associated with set of application features set to a value indicating that the set of application features are activated.

In the above method, the feature customization information includes version information for the diabetes intervention application executing on the computing device. In the above method, the feature customization information includes operating environment information for the computing device and glucose monitoring devices connected with the computing device. In the above method, the access information comprises an access token including the feature customization information and token authentication information. In the above method, the configuration information comprises a mapping of application parameters associated with application features to values corresponding to specific application feature settings.

Certain aspects provide another method for configuring a diabetes intervention application executing on a computing device via a remote server, comprising: receiving, from the diabetes intervention application, a request to initiate a session on the diabetes intervention application, the request including user credentials for a user account associated with a user of the diabetes intervention application; validating that the user credentials are valid for the user account; based on validating that the user credentials are valid, generating access information for the diabetes intervention application to use in retrieving configuration information for the diabetes intervention application from a global configuration service; and transmitting the access information to the diabetes intervention application.

The above method further comprises: receiving, from the computing device, a registration request to create the user account for the user of the diabetes intervention application; retrieving, from the global configuration service, information identifying a plurality of supported regions for the diabetes intervention application based on information included in the registration request; and establishing the user account for the user of the diabetes intervention application including an identification of a region of the plurality of supported regions for the diabetes intervention application.

In the above method, the registration request includes version information for the diabetes intervention application and operating environment information for the computing device and glucose monitoring devices connected with the computing device. In the above method, the information included in the registration request includes an identification of a language to use within the diabetes intervention application. In the above method, establishing the user account for the user of the diabetes intervention application comprises: determining that the identified region is a mismatch to a current geographic location of the computing device included in the registration request; and establishing the user account based on a region of the plurality of supported regions corresponding to the current geographic location of the computing device instead of the identified region.

In the above method, the request to initiate the session on the diabetes intervention application includes version information for the diabetes intervention application. The above method further comprises: comparing the version information for the diabetes intervention application included in the request to version information included in the access information; and based on a comparison of the version information included in the request to version information included in the user account resulting in identification of a mismatch, updating the user account based on the version information included in the request.

In the above method, updating the user account based on the version information included in the request comprises: determining that a feature supported by a version of the application corresponding to the version information included in the user account is deprecated in a version of the application corresponding to the version information included in the request; and transmitting, to the diabetes intervention application, one or more instructions to revert to the version of the application corresponding to the version information included in the user account.

Certain aspects provide yet another method for use by a computing device for configuring a diabetes intervention application executing on the computing device via a remote server, comprising: transmitting, to an authentication service, a request to access features of the diabetes intervention application, the request including user credentials for a user of the diabetes intervention application; receiving, from the authentication service, access information for the diabetes intervention application to use in retrieving configuration information for the diabetes intervention application from a global configuration service, the access information including feature customization information for the diabetes intervention application; transmitting, to the global configuration service, a request for the configuration information, the request including the access information; receiving the configuration information from the global configuration service, the configuration information including a set of assets with which the diabetes intervention application is to be provisioned based, at least in part, on the feature customization information included in the access information; and configuring the diabetes intervention application with the configuration information.

The above method further comprises: transmitting, to the authentication service, a registration request to create the user account for the user of the diabetes intervention application; and receiving, from the authentication service, information identifying a plurality of supported regions for the diabetes intervention application based on information included in the registration request; and transmitting a request to establish the user account, the request including an identification of a region of the plurality of supported regions for the diabetes intervention application.

In the above method, the registration request includes version information for the diabetes intervention application and operating environment information for the computing device and glucose monitoring devices connected with the computing device. In the above method, the information included in the registration request includes an identification of a language to use within the diabetes intervention application. In the above method, the request to access features of the diabetes intervention application includes version information for the diabetes intervention application.

In the above method, the set of assets comprises a plurality of uniform resource locators (URLs) to be used for configuring the diabetes intervention application, the URLs being associated with the feature customization information, and the configuring comprises configuring the diabetes intervention application to access assets using the plurality of URLs for configuring and executing features of the diabetes intervention application. In the above method, the feature customization information includes location information associated with a geographical location of the user of the diabetes intervention application.

In the above method, the feature customization information includes version information for the diabetes intervention application executing on the computing device. In the above method, the feature customization information includes operating environment information for the computing device and glucose monitoring devices connected with the computing device. In the above method, the set of assets comprise one or more feature flags indicating that features associated with the one or more feature flags are activated for use within the diabetes intervention application, the one or more feature flags being associated with the feature customization information, and configuring the diabetes intervention application comprises activating the features associated with the one or more feature flags and disabling features not associated with the one or more feature flags.

Another aspect is an apparatus, comprising: two or more means for performing any one of the above methods.

Another aspect is an apparatus, comprising: at least one processor; and a memory storing code that when executed by the at least one processor causes the apparatus to perform any one of the above methods.

Another aspect is a non-transitory computer readable medium storing computer executable code, comprising: code for performing any one of the above methods.

### BRIEF DESCRIPTION OF THE DRAWINGS

FIG. 1A illustrates an example glucose monitoring system including an application configured via a global configuration service (GCS) and an authentication service, according to some embodiments disclosed herein.
FIG. 1B illustrates the glucose monitoring system of FIG. 1A, in more detail, along with a number of mobile devices, according to some embodiments disclosed herein.
FIG. 2 illustrates an example of a system in which a GCS and an authentication service are used to configure a diabetes intervention application, according to some embodiments disclosed herein.
FIG. 3 is a message flow diagram illustrating messages exchanged between a diabetes intervention application, an authentication service, and a GCS to generate user credentials for an application configured through the GCS, according to some embodiments described herein.
FIG. 4 is a message flow diagram illustrating messages exchanged between a diabetes intervention application, an authentication service, and a GCS to configure the diabetes intervention application, according to some embodiments described herein.
FIG. 5 is a flow diagram illustrating operations performed for authenticating a user of a diabetes intervention application configured through a GCS, such as the GCS of FIG. 1A, FIG. 1B, or FIG. 2, according to some embodiments described herein.
FIG. 6 is a flow diagram illustrating operations performed for initiating and configuring a diabetes intervention application executing on a user device through a GCS and an authentication service, according to some embodiments described herein.
FIG. 7 is a flow diagram illustrating operations performed to configure a diabetes intervention application through a GCS, such as the GCS of FIG. 1A, FIG. 1B or FIG. 2, according to some embodiments disclosed herein.
FIG. 8 is a block diagram depicting a computing device configured to perform the operations of FIGs. 5 through 7, according to certain embodiments disclosed herein.

### DETAILED DESCRIPTION OF CERTAIN INVENTIVE EMBODIMENTS

Typically, applications (e.g., mobile applications) are deployed on a patient's device (e.g., mobile device, such as a smartphone, smart wearable device, or the like) through an application store that may deliver different versions of the same application based on, for example, location information associated with the user, cost considerations, device capabilities, etc. Location information may indicate the location from which the user is accessing the application store (which may not be the user's permanent location) or the user's registered location (e.g., the user's permanent location of residence), etc.

As an example, a safety critical application may be subject to regulation and approval by one or more regulatory authorities. Because some features may not be approved for use in some geographic locations, different versions of the application may exist, with each version including a set of features approved by the regulatory authorities for a specific location in which the application is being deployed. In another example, cost considerations may prompt the deployment of multiple versions of a software application. One version of the software application may include a more limited set of features than another version of the software application, but may be made available at a lower cost. By implementing different sets of features in different versions of the application at different end-user costs, users may be able to select one of a number of versions of the application, and the corresponding features, according to their ability and/or willingness to pay for different sets of features. In still another example, different versions of an application may be deployed to address different use cases. For example, in a diabetes management application, different features may be implemented based on whether the application is being used to treat or manage Type 1 diabetes or Type 2 diabetes; whether the application is being used to treat gestational patients; and the like.

However, maintaining and provisioning different versions of the same application presents many different technical problems and challenges. For example, typically, an application is provisioned by an application provider to users in different locations in the world through an application store. Because of varying specifications in different locations where the application is deployed, however, application providers may maintain different application binary files ("application binaries") for the same application in an application data store and provision the different application binaries to corresponding users in the different locations in order to comply with or comport to the relevant specifications. Note that the varying specifications may include location based and non-location based specifications. Location based specifications may include regulatory guidelines (e.g., regulatory bodies in one location may prohibit the use of certain features), user preferences (e.g., based on research, it may be determined that users in one location may dislike certain features), strategic decisions made by the application provider (e.g., the application provider may determine that certain features should be provided in one location but not another or should be provided as an optional feature that users in one location can choose to enable but is not available to users in another location), technological limitations (e.g., because of limited access to the Internet in a certain location), etc.

Non-location based specifications may include specifications that relate to user-specific information, other than the user's location. For example, certain features may be subscription-based, regardless of the user's location. In another example, certain features may be appropriate based on the use case or goals associated with the app. For example, the use case may relate to the health state of the user, where certain features may be appropriate for treating certain diseases, while other features may be appropriate for other diseases. In another example, the use case may be related to the goals of the app, e.g., disease management, health and wellness, diagnostics, clinical use cases. In yet another example, the app contents may vary according to the nature of the usage of the app, such as the length of use (e.g., limited use, intermittent use, long term use), primary user (e.g. clinician vs. patient), or other usage information that may affect the type of features and capabilities provided through the app. In certain implementations, certain features may be made available according to application cost tiers, subscriptions, etc. Still further, certain features may be made available based on considerations such as user sophistication, preferences, input, or other user or host data.

Typically, each application binary corresponds to a different configuration of the same application, where each configuration corresponds to a unique set of features, feature settings, and/or resources. The unique set of features, feature settings, and resources is hard-coded in each of the different application binaries. To illustrate this with an example, feature X of an application may be preferred by users or approved for use in location A, but not location B. In such an example, application binary A, which includes feature X, is provided to users in location A, while application binary B, which may be similar or identical to application binary A, except for application binary B not having feature X, is provided to users in location B. Thus, different application binaries, associated with different configurations of an application, may be maintained for provisioning to users in different locations. When downloaded and executed on a user's device, each of these different application binaries provide a different configuration of the application. Generally, for each application binary that is maintained by an application provider, a corresponding codebase is also maintained to allow developers to change or update the corresponding version of the application. A codebase comprises higher level code written by developers to create an application.

However, maintaining different application binaries and corresponding codebases for different configurations of the same application may have major technical drawbacks, including resource inefficiency. First, these application binaries and corresponding codebases may significantly overlap in content. As an example, code associated with certain common functionalities of the application may be reused for all of the different configurations of the application. As a result, storing different application binaries and the corresponding codebases for different configurations of the same application means that the overlapping or common code is being stored multiple times, which is storage inefficient. For example, given a number *n* of location-specific application binaries (and the corresponding codebases), the total amount of code stored for these applications may be *n* * commonCodebaseSize + locationSpecificCodebaseSize. In other words, for these *n* location-specific application binaries, the amount of wasted storage space on the storage systems on which the codebases are maintained may be (*n*-1) * commonCodebaseSize.

For an application in which location-specific versions share a significant amount of common code and other assets for location-agnostic functionality, such as login, core calculation and visualization functionality, shared graphical assets, and the like, this may mean that a significant amount of storage space and other compute resources may be needed for building and maintaining the application. Similarly, for the application store, the amount of resources needed to make multiple application binaries available for distribution may be significantly more than the minimum amount of resources that would be needed to store an application including all of the location-specific functionality implemented across the multiple application binaries.

In addition, when certain common code needs to be updated or debugged, developers have to propagate that change to each and every one of the codebases and recompile and deploy application binaries for each of the codebases, which is compute inefficient and increases the amount of work that needs to be performed to maintain the various configurations for the application. Further, if changes to the common code are propagated to only some of the codebases, errors may remain in some application binaries, or some application binaries may remain outdated. While some failures to maintain the application binaries may result in (or fail to solve) minor issues such as inconsistencies in a graphical user interface, other failures may have more severe ramifications. For example, if a security vulnerability is resolved in some application binaries but not resolved in other application binaries, the security vulnerability may still be exploited by malicious actors to exfiltrate sensitive data, inject malicious code for execution, or perform other nefarious activity. In another example, if problems communicating with external sensors are resolved in some application binaries but not resolved in other application binaries, some users of the application may continue to experience connectivity issues, which may cause the application to perform erroneous actions based on erroneous or nonexistent data.

Accordingly, certain embodiments described herein are directed to solving the technical problems described above by deploying a single, unified collection of available features, feature settings, and/or resources of an application. Hereinafter features, feature settings, and/or resources of an application may be referred to as "assets." The collection of assets may be, in certain embodiments, provided as a unified application binary. In some examples, in addition to the collection of assets, a global configuration service (hereinafter "GCS") maintains a mapping that maps different specifications to different sets of assets in the collection of assets. In various embodiments, the GCS uses the mapping to generate configuration information for configuring an application that is downloaded on a user device by mapping specifications associated with the user to a certain subset of assets in the collection of assets. In certain embodiments, the configuration information is then used to configure the application on the user device with the certain subset of assets. As used herein, "features" refer to functionality implemented in an application that may be enabled or disabled based on a user-specific configuration. As used herein, "feature settings" refer to various options that change the functionality of a given feature in the application, such as settings that specify units of measure, user interface appearance options, or the like. As used herein, "resources" refer to code, graphics files, uniform resource locators (URLs) audio files, or other content that can be used by the application during application execution.

For example, as discussed in further detail herein, when a user requests to download the application from an application store, a single collection of assets, for example as a unified application binary, is downloaded to the user's device, regardless of the specifications associated with the user. Such specifications may include a type of device being used by the user for downloading and executing the application, the product packaging associated with the user or the user's analyte monitoring system (e.g., transmitter, sensor, mobile device, receiver, etc.), the set of features subscribed to by the user, the type of user, user disease type or progression, app use case, the region in which the user is located or other user characteristics that may dictate the features, feature settings or assets that should be provided to the user. In one example, when the collection of assets is first downloaded to the user's device, the application is not yet configured based on user-specific specifications. Accordingly, the application sends a configuration request to the GCS, in response to which the GCS maps the user-specific specifications associated with the user to a set of assets in the collection of assets and transmits configuration information to the application that is indicative of the mapped set of assets. Using the configuration information, the application configures itself with the corresponding set of assets. For example, the application activates the set of assets indicated by the configuration information and deactivates or disables the rest of the assets in the application binary. In one example, the application may automatically configure itself with features in the set of assets indicated by the configuration information. Further, the application may automatically configure certain settings of those features as part of the configuration. Additionally, the application may automatically configure the resources to be accessible to the user according to the configuration information.

Storing, maintaining, and provisioning a single, unified application to different users, regardless of user-specific specifications leads to significant resource efficiency. More specifically, a large amount of storage resources may be freed and used for other purposes if a single application binary and codebase are stored and maintained, as opposed to many different ones with overlapping code. In addition, significantly less computing resources may be spent when updating, compiling, and debugging a single application binary and a single codebase, as opposed to many application binaries and codebases. Further, maintaining a single codebase reduces the likelihood of introducing errors into the code during code updates or resolving extant errors in some, but not all, versions of the application.

Further, as discussed above, some applications may be safety critical applications for which regulatory approval is needed before features are made available to users in a given location (e.g., to ensure that the new features do not harm patients using the software application, that the new features comply with security requirements or best practices, etc.). New features in a safety critical application may be approved in some regions earlier than in other regions. Thus, in deploying a new feature in a safety critical application, a developer can choose to deploy different versions of an application, with versions including the new feature being deployed in regions where regulatory approval has been obtained and versions excluding the new feature being deployed in regions where regulatory approval has not been obtained. When multiple codebases exist for multiple versions of an application, additional complexity may be introduced by adding new features in a piecemeal fashion (e.g., as features are approved by the relevant regulatory authorities).

The availability of multiple versions of an application may also complicate user selection of the appropriate application to install and use as well. For example, different versions of an application may use the same (or similar) application icons and thumbnails in an application repository and may have the same (or similar) package names. This may lead to user confusion in selecting the appropriate version of the application; user selection of a version of the application that is inappropriate for the user (which the user may not discover until trying to use the application); user selection of a version of the application that is incompatible with one or more user devices; and the like.

An example of an application that may be maintained, provisioned, and configured by the GCS, according to the embodiments described herein, is a diabetes intervention application. In one example, as described herein, the diabetes intervention application delivers guidance, which may assist patients, caregivers, healthcare providers, or other users in the management of diabetes. Accordingly, certain aspects of an application are described herein with respect to a diabetes intervention application as an example, but it should be noted that the techniques described herein are also applicable to other suitable types of applications including applications that provide health related information or health related guidance to a user. Diabetes intervention applications such as those described herein may deliver guidance with respect to lifestyle or clinical/patient outcomes by meeting a variety of challenges, such as overnight glucose control (e.g., reduce incidence of hypoglycemic events or hyperglycemic excursions), glucose control during and after meals (e.g. use historical information and trends to increase glycemic control), hyperglycemia corrections (e.g., increase time in target zone while avoiding hypoglycemic events from over-correction), hypoglycemia treatments (e.g., address hypoglycemia while avoiding "rebound" hyperglycemia), exercise, and/or other health factors. In certain embodiments, the application may further be configured with optimization tools that learn a patient's physiology and behavior and calculate guidance to help the patient identify optimal or desirable therapy parameters, such as basal insulin requirements, insulin to carbohydrate ratios, correction factors, and/or changes to insulin sensitivity due to exercise.

In certain embodiments, to provide relevant and effective guidance, the application utilizes input from one or more physiological sensors, such as one or more analyte sensors. An example of an analyte sensor described herein is a glucose monitoring sensor that measures a concentration of glucose and/or a substance indicative of the concentration or presence of glucose and/or another analyte in the user's body. In some embodiments, the glucose monitoring sensor is a continuous glucose monitoring device, for example a subcutaneous, transdermal, transcutaneous, intraocular and/or intravascular (e.g., intravenous) device. The glucose monitoring sensor can use any method of glucose measurement, including enzymatic, chemical, physical, electrochemical, optical, optochemical, fluorescence-based, spectrophotometric, spectroscopic (e.g., optical absorption spectroscopy, Raman spectroscopy, etc.), polarimetric, calorimetric, iontophoretic, radiometric, and the like.

The glucose monitoring sensor can use any known detection method, including invasive, minimally invasive, and non-invasive sensing techniques, to provide a data stream indicative of the concentration of the analyte in a host. The data stream typically incudes a stream of raw data signals used to provide useful analyte values, such as a patient or health care professional (HCP, e.g., doctor, physician, nurse, caregiver), who may be using the sensor.

In some embodiments, the glucose monitoring sensor is an implantable sensor, such as described with reference to U.S. Pat. No. 6,001,067 and U.S. Patent Publication No. US-2011-0027127-A1. In some embodiments, the glucose monitoring sensor is a transcutaneous sensor, such as described with reference to U.S. Patent Publication No. US-2006- 0020187-A1. In some embodiments, the glucose monitoring sensor is a dual electrode analyte sensor, such as described with reference to U.S. Patent Publication No. US-2009-0137887- A1. In some embodiments, the glucose monitoring sensor is configured to be implanted in a host vessel or extracorporeally, such as the sensor described in U.S. Patent Publication No. US-2007-0027385- A1. These patents and publications are incorporated herein by reference in their entirety.

Although much of the description and examples below are described in relation to a diabetes intervention application, the system and methods of the embodiment described herein may be used in conjunction with any health-related application that is provided to the user to improve the user's health. For example, a health-related application may help the user with treating a certain disease or just help with improving the health of a user who is not necessarily diagnosed with a disease.

### EXAMPLE SYSTEM

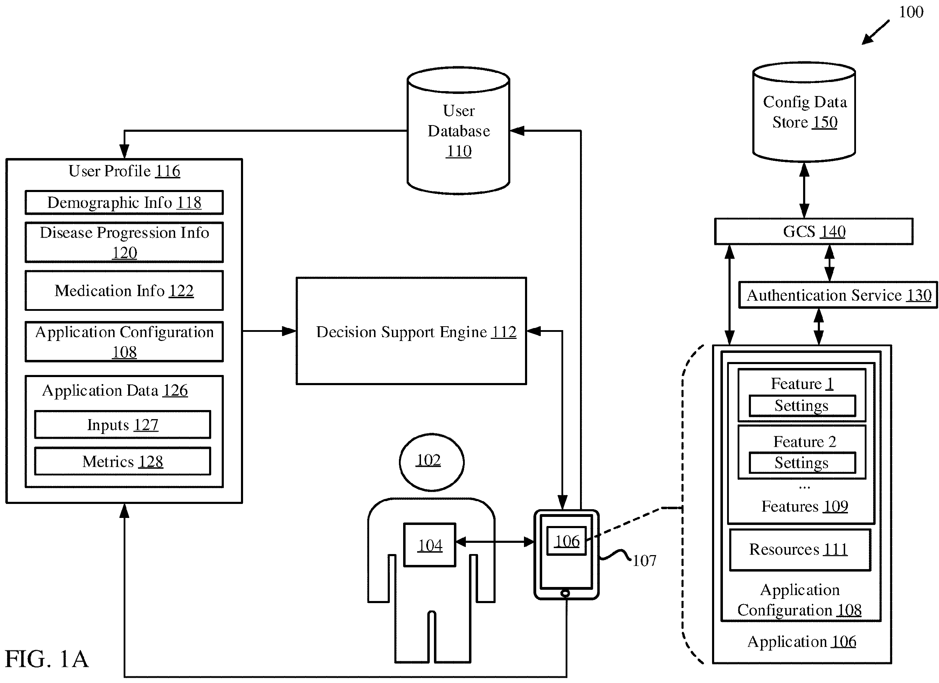

FIG. 1A illustrates an example system 100 for maintaining, provisioning, and configuring application 106, which monitors a condition of, provides decision-support guidance to, and/or determines or directs administration of one or more treatments for user 102 (hereinafter "the user"). The user, in certain embodiments, may be the patient or the patient's caregiver, healthcare provider or other entity that monitors patient health parameters. In the embodiments described herein, the user is assumed to be the patient for simplicity only but is not so limited. System 100 includes a glucose monitoring system 104, a mobile device 107 that executes application 106, a decision support engine 112, a user database 110, an authentication service 130, a GCS 140, and a config data store 150.

In certain embodiments, glucose monitoring system 104 includes a sensor electronics module and a glucose sensor that measures a concentration of blood glucose and/or a substance indicative of the concentration or presence of glucose and/or another analyte in the user's body. In certain embodiments, the glucose sensor is configured to perform measurements on a continuous basis. The sensor electronics module transmits the blood glucose measurements to mobile device 107 for use by application 106. In some embodiments, the sensor electronics module transmits the glucose measurements to mobile device 107 through a wireless connection (e.g., Bluetooth connection). In certain embodiments, mobile device 107 is a smart phone. However, in certain embodiments, mobile device 107 may instead be any other type of computing device such as a laptop computer, a smart watch, a tablet, or any other computing device capable of executing application 106.

While system 100 illustrates a glucose monitoring system 104, it should be recognized that the embodiments described herein are similarly applicable to any other type of physiological monitoring systems that may be used, for example, as part of system 100 to monitor and/or treat a patient. Example physiological monitoring systems may include electrical or optical heart rate monitoring systems, pulse oximeters, temperature monitors, blood pressure monitors, hydration monitors, various analyte sensors (e.g., lactate sensor, ketone sensor, etc.), and so on. Further, it should be recognized that glucose monitoring system 104 or other physiological monitoring systems may include one or more sensors providing input into such systems.

In certain embodiments, decision support engine 112 refers to a set of software instructions with one or more software modules. In some embodiments, decision support engine 112 executes entirely on one or more computing devices in a private or a public cloud. In such embodiments, application 106 communicates with decision support engine 112 over a network (e.g., Internet). In some other embodiments, decision support engine 112 executes partially on one or more local devices, such as mobile device 107, and partially on one or more computing devices in a private or a public cloud. In some other embodiments, decision support engine 112 executes entirely on one or more local devices, such as mobile device 107.

In certain embodiments, decision support engine 112 is configured to process a set of inputs received from application 106 and compute a plurality of metrics 128, which can then be stored in user profile 116. Inputs 127 and metrics 128 may be used by application 106, such as by different features of application 106, to provide real-time guidance and treatments to the user. The various data points of user profile 116 are described in further detail below. In certain embodiments, user profiles, including user profile 116, are stored in a user database 110, which is accessible to application 106 as well as decision support engine 112 over one or more networks (not shown). User database 110, in some embodiments, refers to a storage server that may operate in a public or private cloud.

The real-time guidance and treatments provided to the user help improve the user's physiological conditions and/or enable the user with making more informed decisions. To provide effective, relevant, and on-time guidance and treatments to the user, in certain embodiments, a feature of application 106 may take as input information relating to the user, which is stored in user profile 116, and/or information relating to a pool of similar users, which is stored in user profiles of such users in user database 110. In certain embodiments, a feature of application 106 may interact with the user through various ways such as text, email, notifications (e.g., push notifications), phone calls, and/or other forms of communication such as displaying content (e.g., graphs, trends, charts, etc.) on the user interface of application 106.

As shown in FIG. 1A, application 106 is uniquely configured with a certain configuration, which is logically represented as application configuration 108. Application configuration 108 corresponds to a set of features 109 and resources 111 that are active and available for use by a user of application 106. In certain embodiments, each of features 109 is configured to monitor a condition of, provide decision-support guidance to, and/or determine/administer one or more treatments for user 102. As shown, a feature (e.g., features 1 and 2) may also comprise a setting that defines the operations of the feature. In certain embodiments, changing a feature's setting may result in changing the way the feature operates, such as the way the feature provides guidance and interacts with the user. For example, changing a feature's setting may result in, among other things, changing the feature's unit of measurement for calculating and displaying information to a user (e.g., pounds versus kilos). Resources 111 may also include uniform resource locators (URLs) pointing to remote resources and other types of resources.

Features 109 and resources 111 represent subsets of larger sets of assets. The larger sets of assets represent all the features, feature settings, and/or resources that are included in the application 106's binary file and/or provisioned through GCS 140. In the example of FIG. 1A, the remaining assets (of the larger sets of assets) are deactivated (e.g., not configured) for application 106. In other words, a user of application 106 is not able to access or use the remaining assets of application 106. Although not limited to this list, some example features may include one or more of an objective setting and identification feature, reward features, reporting features, behavioral intervention features, exercise management feature, medication reminder features, a glycemic impact estimator feature, an educational feature, on-boarding features, application logging features, etc.

As described in further detail below, application 106 is configured with application configuration 108, based on configuration information received from GCS 140 (and retrieved from config data store 150), after application 106 is downloaded on mobile device 107 by the user. Generally, application 106 may begin from an uninitiated state (e.g., on initial launch after a user downloads the application binary for application 106, after a session times out, etc.). From this uninitiated state, application 106 may present a login screen to a user of application 106. If the user has account credentials that allow the user to use and access features in application 106, the user can enter those credentials into the login screen and submit the credentials to authentication service 130 in order to authenticate the user and initiate an active session in the application. Otherwise, as discussed in further detail below, the user can request that authentication service 130 create credentials that will allow the user to use the application 106.

Authentication service 130 generally uses user identification information to authenticate or validate a user and provide access information to application 106 that application 106 can use to obtain configuration information from GCS 140. In some examples, authentication service 130 validates the user credentials (e.g., user ID and password/passkey) provided by a user through the login screen of application 106 and, if the user credentials are validated as belonging to an active account, authentication service 130 generates and returns access information for application 106 to use to configure the application 106 through GCS 140. Generally, the access information generated by authentication service 130 includes user identity-related information (e.g., information indicating that the user has been authenticated by authentication service 130, such as a cryptographic hash, a magic number, or the like) and feature customization information. In some aspects, the access information returned by authentication service 130 may comprise or be structured as an access token.

The feature customization information may indicate user specifications and may include, for example, version information for the application 106, location information for the user (which may be determined based on user specification of the user's location or based on geolocation information from mobile device 107), the type of user or user disease, operating environment information for the mobile device 107, type of mobile device 107, user subscription tier, information about application 106 and/or glucose monitoring system 104, various device and/or sensor codes associated with the user, and other information that can be used to uniquely identify a user and aid in identifying an application configuration 108 that is appropriate for the user. In some aspects, where the access information is received in the form of an access token, the access token may also include information that can be used to validate that the token is authentic (e.g., was validly generated by authentication service 130 and not generated by an imposter service). For example, the access token may include a validity period after which application 106 may require that the user 102 re-authenticate with authentication service 130 to continue using the application 106, cryptographic information used to authenticate the token, or the like.

After receiving the access information from authentication service 130, application 106 may request application configuration 108 for the user of application 106 from GCS 140. Generally, the request for configuration information includes the access information (e.g., an access token) received from authentication service 130. GCS 140 can determine whether the access token is valid. If the access information is determined to be valid, GCS 140 can then generate and transmit configuration information for application 106 such that application 106 can be configured with assets that are enabled for the user based on the feature customization information. The configuration information may include, for example, flags corresponding to various features and/or feature settings of application 106, resource identifiers (e.g., uniform resource locators (URLs) pointing to remote resources that implement the activated features), or the like. The configuration information may be generated by GCS 140 based on configuration rules and other information stored, for example, in config data store 150. These configuration rules, which were previously referred to as a mapping, map feature customization information associated with a user to a set of assets (i.e., various features and/or feature settings of application 106, resource identifiers, etc.). In other words, the GCS 140 maps feature customization information associated with a user to a set of assets, using the configuration rules, and then generates configuration information that indicates the mapped set of assets.

Application 106 then uses the received configuration information to configure the application by activating the various features and/or feature settings and pointing to remote resources identified in the application configuration information. The application 106 may then be ready for use to obtain data from glucose monitoring system 104 and/or other resources (e.g., accelerometers or location information devices in or connected with mobile device 107, data entry from mobile device 107, etc.) to monitor the user's blood glucose measurements and take various actions in response to the user's blood glucose measurements.

In some examples, if a user does not have credentials allowing the user to use application 106, application 106 directs the user to a credential creation workflow provided by authentication service 130. The authentication service 130 may request that the user provide contact information and information identifying a location associated with the user, which may be manually entered or determined based on location information devices (e.g., satellite positioning systems such as NAVSTAR GPS, GLONASS, or GALILEO; IP address geolocation, etc.) integrated into or otherwise connected with mobile device 107. Authentication service 130 may then authenticate the user's contact information, and upon authentication, continue with the process of generating account credentials for the user.

In generating account credentials for the user, authentication service 130 may obtain a list of supported locations from GCS 140 (or a data store associated therewith, such as config data store 150) and display the list of supported locations to the user for selection. In some aspects, authentication service 130 may automatically select a location from the list of supported locations based on user-provided information identifying the location associated with the user (as discussed above) or based on a location of the mobile device 107 determined based on one or more location information or identification devices or services in or connected with mobile device 107. The automatically selected location, however, may be overridden by user selection of another one of the supported locations. In some aspects, authentication service 130 can determine that a determined location of the user's mobile device 107 is a mismatch with user-provided location information. In such a case, authentication service 130 can override the user-provided location information with the determined location of the user's mobile device 107 and proceed with establishing a user account based on the determined location of the user's mobile device 107. After creating the appropriate credentials (e.g., username, password, location, and other information that may be requested by authentication service 130), authentication service 130 can create an account including the created credentials for the user and generate an access token to be used by application 106 to configure the application for the user, as discussed above.

As discussed, GCS 140 is generally configured to receive requests to configure an application 106 and provide, to the requesting devices, configuration information generated based on access information, such as an access token, included in the received requests. Upon receiving the access information from an application 106, GCS 140 can attempt to validate the access information. In some aspects, the access information may be encrypted using a cryptographic key known to the authentication service 130 and the GCS 140. If the GCS 140 is thus unable to decrypt the access information (e.g., recovers nonsensical information after an attempt to decrypt the token), GCS 140 can determine that the access information is invalid, and configuration of the application 106 may be terminated. In another aspect, the access information may include a validity identifier that GCS 140 can use to authenticate the access information. The validity identifier may be data that GCS 140 can derive from other information in the access information or may be information that GCS 140 can use to request validation of the access information from authentication service 130. It should be noted, however, that various techniques can be used to determine the validity of the access information, and correspondingly, whether the application 106 can be configured for the user.

If GCS 140 determines that the received access information is, in fact, valid, configuration service can use the received access information to generate configuration information for use in configuring application 106. For example, GCS 140 can use some or all of the feature customization information included in the received access information, such as location information (e.g., the location information associated with the user), version information of the application 106 from which the configuration request was received, information about the operating environment in which application 106 is being used (e.g., information about sensors being used to generate data analyzed by application 106, the operating system of the mobile device 107 on which application 106 is being used, the type of the mobile device 107, etc.), and/or other information, to identify configuration options to activate and/or deactivate for the user. The configuration information may include flag settings identifying which flags, corresponding to features and/or feature settings in the application 106, to activate or deactivate, information identifying the location of remote resources that application 106 can use, and other information that configures the operations of application 106.

In some aspects, GCS 140 can use the version information included in the access information and the version information of the application 106, from which the configuration request was received, to perform various actions with respect to the user's account. For example, if GCS 140 determines that there is a mismatch between the version information included in the access information and the version information for the application 106, GCS 140 can update the user account to reflect that the user is using a new (or different) version of application 106. In another example, GCS 140 can use the version information included in the access information and the version information of the application 106 to determine whether features of the version of application 106 specified in the access token are deprecated in the version of the application 106 from which the configuration request was received. If a feature is deprecated in the version of the application 106 from which the configuration request was received, GCS 140 can transmit, to application 106, instructions to revert to a previous version of the application in which the feature is not deprecated. For example, GCS 140 can instruct application 106 to revert to the version of the application identified in the access token.

As described above, in certain embodiments, application 106 is configured to take as input information relating to the user and store the information in a user profile 116 of the user. For example, application 106 may obtain and record the user's demographic info 118, disease progression info 120, and/or medication info 122 in user profile 116. In certain embodiments, demographic info 118 may include one or more of the user's age, BMI (body mass index), ethnicity, gender, etc. In certain embodiments, disease progression info 120 may include information about the user's disease, such as whether the user is Type 1, Type 2, or pre-diabetes or whether the user has gestational diabetes. In certain embodiments, information about the user's disease may also include the length of time since diagnosis, the level of diabetes control, level of compliance with diabetes management therapy, predicted pancreatic function, other types of diagnosis (e.g., heart disease, obesity) or measures of health (e.g., heart rate, exercise, stress, sleep, etc.), and/or the like. In certain embodiments, medication info 122 may include information about the amount and type of insulin or non-insulin diabetes medications and/or non-diabetes medication taken by the user.

In certain embodiments, application 106 may obtain demographic info 118, disease progression info 120, and/or medication info 122 from the user in the form of user input or from other sources. In certain embodiments, as some of this information changes, application 106 may receive updates from the user or other sources. In certain embodiments, user profiles, including user profile 116, are stored in a user database 110, which is accessible to application 106 as well as decision support engine 112 over one or more networks (not shown). User database 110, in some embodiments, refers to a storage server that may operate in a public or private cloud.

In certain embodiments, in addition to the user's demographic info 118, disease progression info 120, disease type, and/or medication info 122, application 106 obtains an additional set of inputs 127 that are also utilized by features 109 to provide output to the user. In certain embodiments, such inputs 127 are obtained on a continuous basis. In certain embodiments, application 108 receives inputs 127 through user input and/or a plurality of other sources, including glucose monitoring system 104, other applications running on mobile device 107, and/or one or more other sensors and devices. These inputs may include, for example, device identifying information (e.g., a serial number which can be mapped to a specific device model), sensor identifying information, firmware version information for connected devices or sensors, etc. In certain embodiments, such sensors and devices include one or more of, but are not limited to, an insulin pump, other types of analyte sensors, sensors or devices provided by mobile device 107 (e.g., accelerometer, camera, global positioning system (GPS), heart rate monitor, etc.) or other user accessories (e.g., a smart watch), or any other sensors or devices that provide relevant information about the user.

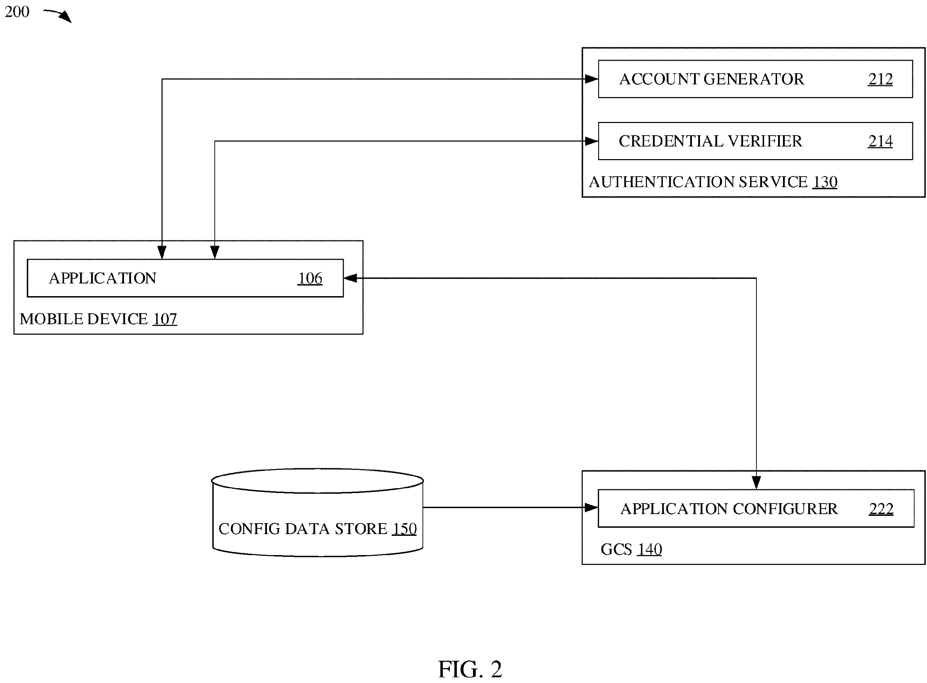

In certain embodiments, application 106 further uses at least part of inputs 127 to obtain a plurality of metrics 128, such as behavioral metrics or outcome metrics. As further described in relation to FIG. 2, in some embodiments, application 106 transmits at least part of inputs 127 to decision support engine 112 for processing, based on which decision support engine 112 generates the plurality of metrics. In certain embodiments, metrics 128 may then be used by application 106 as input to features 109 for providing output to the user. In certain embodiments, metrics 128 are also stored in user profile 116, which may be stored for future analysis, used to adapt application configuration 108, or the like.

In certain embodiments, metrics 128 may include behavioral data that may be indicative of the user's behaviors and habits, such as one or more of the user's behavior or interaction with respect to the application, the user's behavior with respect to the treatment of the disease, health-related behaviors, etc. Behavioral metrics may include real-time metrics, past metrics, and/or trends. Outcome metrics may, at lease in some cases, be generally indicative of the user's health or state, such as one or more of the user's physiological or psychological state (e.g., stress level, happiness, etc.), trends associated with the user's health or state, how far or close the user is to meeting their objectives based on the user's health or state, etc. In certain embodiments, outcome metrics may be directly correlated with the behavioral metrics, meaning that outcome metrics may be the result of the user's behavior or pattern of behaviors. Examples of outcome metrics include one or more of metrics associated with metabolic rates, glucose levels and trends, the user's health or sickness, etc. In certain embodiments, outcome metrics may include real-time metrics, past metrics, and/or trends.

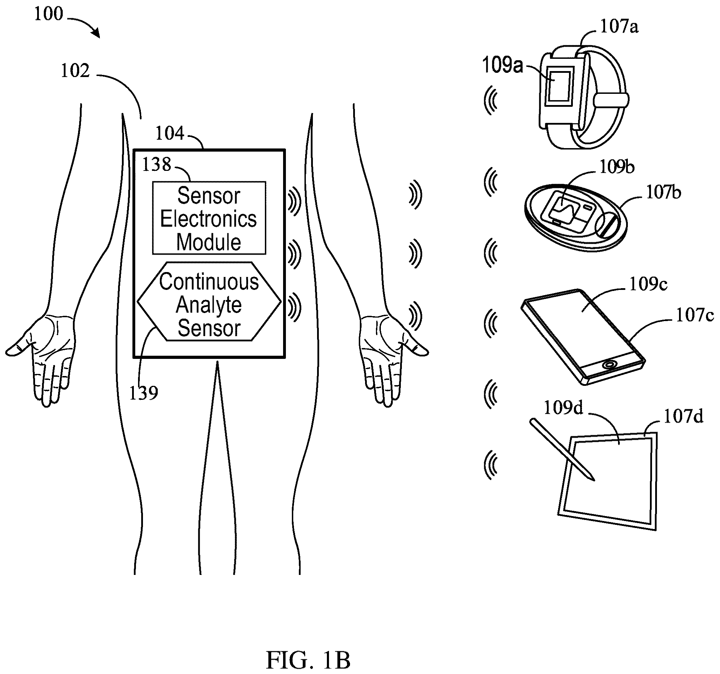

FIG. 1B illustrates glucose monitoring system 104 in more detail. FIG. 1B also illustrates a number of mobile devices 107a, 107b, 107c, and 107d. Note that mobile device 107 of FIG. 1A may be any one of mobile devices 107a, 107b, 107c, or 107d. In other words, any one of mobile devices 107a, 107b, 107c, or 107d may be configured to execute application 106. Glucose monitoring system 104 may be communicatively coupled to mobile devices 107a, 107b, 107c, and/or 107d.

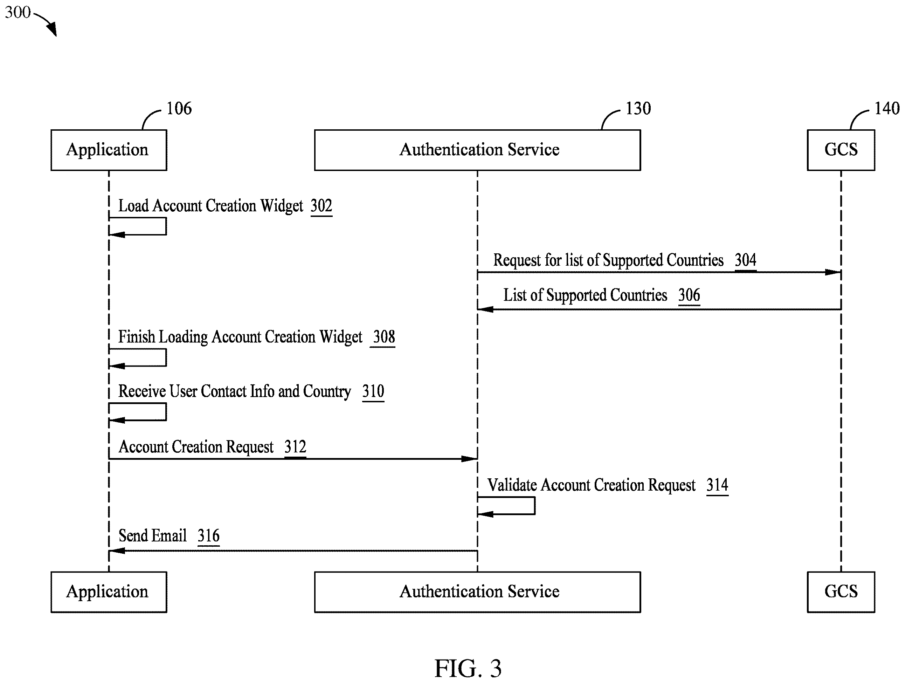

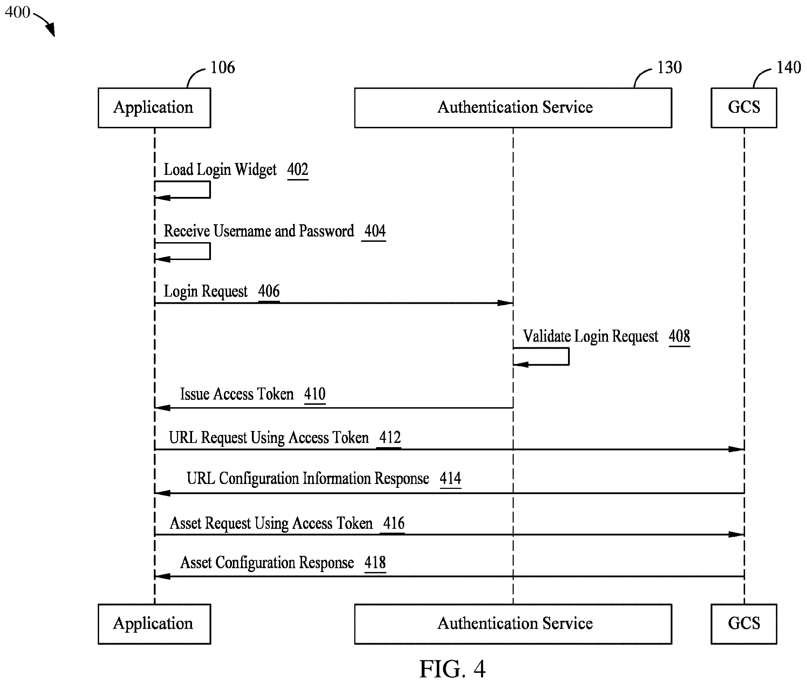

By way of an overview and an example, glucose monitoring system 104 may be implemented as an encapsulated microcontroller that makes sensor measurements, generates analyte data (e.g., by calculating values for continuous glucose monitoring data), and engages in wireless communications (e.g., via Bluetooth and/or other wireless protocols) to send such data to remote devices, such as mobile devices 107a, 107b, 107c, and/or 107d. Paragraphs [0137]-[0140] and FIGs. 3A, 3B, and 4 of U.S. App. No. 2019/0336053 further describe an on-skin sensor assembly that, in certain embodiments, may be used in connection with glucose monitoring system 104. Paragraphs [0137]-[0140] and FIGs. 3A, 3B, and 4 of U.S. App. No. 2019/0336053 are incorporated herein by reference.

In certain embodiments, glucose monitoring system 104 includes an analyte sensor electronics module 138 and a glucose sensor 139 associated with analyte sensor electronics module 138. In certain embodiments, analyte sensor electronics module 138 includes electronic circuitry associated with measuring and processing analyte sensor data or information, including algorithms associated with processing and/or calibration of the analyte sensor data/information. Analyte sensor electronics module 138 may be physically/mechanically connected to glucose sensor 139 and can be integral with (i.e., non-releasably attached to) or releasably attachable to glucose sensor 139.

Analyte sensor electronics module 138 may also be electrically coupled to glucose sensor 139, such that the components may be electromechanically coupled to one another. Analyte sensor electronics module 138 may include hardware, firmware, and/or software that enable measurement and/or estimation of levels of the analyte in the user via glucose sensor 139 (e.g., which may be/include a glucose sensor). For example, analyte sensor electronics module 138 can include one or more potentiostats, a power source for providing power to glucose sensor 139, other components useful for signal processing and data storage, and a telemetry module for transmitting data from the sensor electronics module to one or more display devices. Electronics can be affixed to a printed circuit board (PCB) within glucose monitoring system 104, or platform or the like, and can take a variety of forms. For example, the electronics can take the form of an integrated circuit (IC), such as an Application-Specific Integrated Circuit (ASIC), a microcontroller, a processor, and/or a state machine.

Analyte sensor electronics module 138 may include sensor electronics that are configured to process sensor information, such as sensor data, and generate transformed sensor data and displayable sensor information. Examples of systems and methods for processing sensor analyte data are described in more detail herein and in U.S. Pat. Nos. 7,310,544 and 6,931,327 and U.S. Patent Publication Nos. 2005/0043598, 2007/0032706, 2007/0016381, 2008/0033254, 2005/0203360, 2005/0154271, 2005/0192557, 2006/0222566, 2007/0203966 and 2007/0208245, all of which are incorporated herein by reference in their entireties.

Glucose sensor 139 is configured to measure a concentration or level of the analyte in the user 102. The term analyte is further defined by paragraph [0117] of U.S. App. No. 2019/0336053. Paragraph [0117] of U.S. App. No. 2019/0336053 is incorporated herein by reference. In some embodiments, glucose sensor 139 comprises a continuous glucose sensor, such as a subcutaneous, transdermal (e.g., transcutaneous), or intravascular device. In some embodiments, glucose sensor 139 can analyze a plurality of intermittent blood samples. Glucose sensor 139 can use any method of glucose-measurement, including enzymatic, chemical, physical, electrochemical, spectrophotometric, polarimetric, calorimetric, iontophoretic, radiometric, immunochemical, and the like. Additional details relating to a continuous glucose sensor are provided in paragraphs [0072]-[0076] of U.S. App. No. 13/827,577. Paragraphs [0072]-[0076] of U.S. App. No. 13/827,577 are incorporated herein by reference.

With further reference to FIG. 1B, mobile devices 107a, 107b, 107c, and/or 107d can be configured for displaying (and/or alarming) displayable sensor information that may be transmitted by sensor electronics module 138 (e.g., in a customized data package that is transmitted to the display devices based on their respective preferences). Each of mobile devices 107a, 107b, 107c, and/or 107d may respectively include a display such as touchscreen display 109a, 109b, 109c, and/or 109d for displaying a graphical user interface of application 106 for presenting sensor information and/or analyte data to user 102 and/or receiving inputs from user 102. In certain embodiments, the mobile devices may include other types of user interfaces such as voice user interface instead of or in addition to a touchscreen display for communicating sensor information to user 102 of the mobile device and/or receiving user inputs. In certain embodiments, one, some, or all of mobile devices 107a, 107b, 107c, and/or 107d may be configured to display or otherwise communicate the sensor information as it is communicated from sensor electronics module 138 (e.g., in a data package that is transmitted to respective display devices), without any additional prospective processing required for calibration and/or real-time display of the sensor data.

The plurality of mobile devices 107a, 107b, 107c, and/or 107d depicted in FIG. 1B may include a custom or proprietary display device, for example, analyte display device 107b, especially designed for displaying certain types of displayable sensor information associated with analyte data received from sensor electronics module 138 (e.g., a numerical value and/or an arrow, in certain embodiments). In certain embodiments, one of the plurality of mobile devices 107a, 107b, 107c, and/or 107d includes a smartphone, such as mobile phone 107c, based on an Android, iOS, or another operating system configured to display a graphical representation of the continuous sensor data (e.g., including current and/or historic data).

FIG. 2 illustrates an example of a computing system 200 in which GCS 140 and authentication service 130 are used to configure application 106 (e.g., diabetes intervention application), according to some embodiments disclosed herein. As illustrated, computing system 200 includes a mobile device 107, authentication service 130, GCS 140, and a config data store 150.

Authentication service 130 generally represents a service through which users of application 106 can create credentials for using the application 106 and request access to the application 106. As illustrated, authentication service 130 includes an account generator 212 and a credential verifier 214.

Account generator 212 is configured to generate user credentials in response to a request to create an account for a user received from application 106. In some aspects, the request may include contact information for the user and other information that can be used to validate the user and the user's contact information. Upon receiving the request to create the account, account generator 212 can provide information about the supported locations for application 106 as part of a process to complete user registration and credential generation for the user. Upon completion of the credential creation process, account generator 212 can persist the user's account information to a data store (not shown) for use in subsequently authenticating a user of the application 106.

FIG. 3 is a message flow diagram 300 illustrating an example of messages exchanged between an application 106, authentication service 130 (and more specifically, account generator 212), and GCS 140 to generate user credentials for an application configured through the GCS, according to some embodiments described herein.

As illustrated, to generate user credentials for the diabetes intervention application, the application can load 302 an account creation widget. In loading the account creation widget, the application can provide, to authentication service 130, information about the version of the application, and the authentication service 130 can transmit a request 304 for a list of supported locations (e.g., countries) for the version of the application from the GCS 140. In response, the GCS 140 provides the list of supported countries 306 to the authentication service 130, and the authentication service 130 can provision a location selector in the account creation widget with the list of supported countries received from the GCS. After receiving the list of supported countries, the application 106 completes loading 308 the account creation widget.

After receiving user input 310, including at least user contact information and a selection of one of the supported countries, the application 106 transmits a request 312 to create a user account including the user input to the authentication service 130. The authentication service 130 validates 314 the user contact information and transmits a message 316 to the user including, for example, a link to complete user registration within or outside of the application. Further, once validated, the authentication service can persist user information to a user data store which the authentication service can subsequently access to process login requests and generate access information, such as access tokens, that the application can use to request configuration information from the GCS, as discussed herein.

Credential verifier 214 generally is configured to verify a user's credentials and provide access information (e.g., an access token) to application 106 for use in requesting configuration information from GCS 140. As illustrated, credential verifier 214 may receive a login request from application 106, which initiates a process of authenticating the user of application 106 and generating access information for the user. The login request may include a username and password (or other access code) generated when the user created the user account and the associated user credentials through account generator 212, as discussed above. If the username and password (or other access code) match an entry in a user account repository, credential verifier 214 can determine that the login request was a valid login request and generate access information for the user. The generated access information generally indicates that the user is validly logged into the user account and can initiate a session in application 106. In some aspects, the access information may have a timeout period, after which the user may be requested to provide the user's credentials to credential verifier 214 again in order to generate a new set of access information or renew an already issued set of access information.

Credential verifier 214 may generate access information for the user based on various user specifications that may be stored in a user account repository or received from application 106 (e.g., in the login request received from application 106). For example, credential verifier 214 can generate access information including feature customization information. This feature customization information may be generated based on the various user specifications, such as user identifying information and attributed (e.g., user-provided locality information indicating the country in which the user resides, user disease, user preferences, type of user, etc.), information about mobile device 107 and the version of application 106 executing on mobile device 107 (e.g., major version number, build number, or other information identifying the specific version of the application 106 from which the login request was received), information about the operating environment in which application 106 is executing, information about one or more sensors (e.g., glucose sensor system 104) used by the user, and other information that can be used by GCS 140 to configure application 106.

In some aspects, credential verifier 214 can encrypt the access information using a cryptographic key (or a set of cryptographic keys) known to authentication service 130 and GCS 140 to allow configuration service to validate the access information. In some aspects, credential verifier 214 may generate other information that can be used to verify the access information, such as a cryptographic hash generated based on the access information (e.g., carried in an access token), and include the generated information in the access information for use by configuration service 220 in verifying the access information. For example, credential verifier 214 may concatenate the username, a timestamp associated with the login request (e.g., the time at which the login request was received, an expiration time calculated from the time at which the login request was received, etc.), and location information associated with the user, and generate a verification code based on a cryptographic hash of the concatenated username, timestamp, and location information. It should be recognized, however, that this is only one example of the generated information that credential verifier 214 can generate for use in validating the access information, and that other information and other techniques for generating data for use in validating the access information can be used.

GCS 140 generally represents a service through which application 106 is configured based on access information provided by authentication service 130. As illustrated, GCS 140 includes an application configurer 222.

Application configurer 222 generates configuration information for application 106 based on a received configuration request and received access information. The configuration request may be received, for example, after an application is launched or updated at mobile device 107. To configure the application 106, application configurer 222 can first determine whether the configuration request is valid based on whether the access information is valid. For example, where the access information is received as an access token, application configurer 222 can determine whether the received access token is a valid access token by attempting to decrypt the access token using an *a priori* defined cryptographic key (or set of cryptographic keys).

Because only the correct cryptographic key (or set of cryptographic keys) allows for intelligible data to be retrieved from encrypted data, application configurer 222 can determine whether the access token is valid by attempting to decrypt the access token and determining whether the decrypted access token includes intelligible data. The determination of whether the decrypted access token includes intelligible data may include, for example, searching for one or more defined strings in the decrypted access token, identifying a special value in the decrypted access token, or the like. If application configurer 222 determines that the access token was successfully decrypted, application configurer 222 can proceed to generate configuration information for use by application 106, as discussed in further detail below. Otherwise, application configurer 222 can transmit an error message to application 106 indicating that validation of the access token failed.

In another example, application configurer 222 can determine whether the access information is valid by attempting to match an authentication code included in the access information with a version of the authentication code generated from data included in the access information. In such a case, application configurer 222 may be configured to generate authentication codes using the same techniques (e.g., same algorithms) used by credential verifier 214 to generate authentication codes, as discussed above. If the authentication code included in the received access information matches the version of the authentication code generated by application configurer 222, application configurer 222 can determine that the access information is valid and can proceed to generate configuration information for use by application 106. Otherwise, application configurer 222 can transmit an error message to application 106 indicating that validation of the access information failed.

To generate configuration information for application 106, application configurer 222 can retrieve information from config data store 150 and use the feature customization information included in the access information to identify the assets that are to be enabled for the user of application 106. For example, where the feature customization information includes user location information, application configurer 222 can search config data store 150 for configuration rules or mappings identifying assets that are supported for users located in the location associated with the user from which the configuration request and access token was received. These configuration rules may dictate which flags are to be enabled or indicated as enabled in the configuration information, as well as resource information pointing to resources that application 106 are to access during execution. As an example, a configuration rule may dictate that for a user in Germany, feature X may be activated but feature Y may be deactivated. In such an example, the configuration information generated for the user by the application configurer 222 includes a flag indicating that feature X should be activated for the user.

Flags or other information to enable assets in application 106 may include flags indicating features and feature settings. For example, a flag may indicate that an exercise management feature should be enabled for the user. In another example, a flag may indicate that a certain feature setting of the exercise management feature should be enabled. In yet another example, a flag may indicate whether a feature setting, such as a unit of measure, should be set to the metric system or the imperial system. For example, a first value of a units of measure flag may indicate that the application 106 or a certain feature of the application 106 is to interpret sensor data and generate measurements based on the metric system, while a second value of the units of measure flag may indicate that the application 106 or a certain feature of the application 106 is to interpret sensor data and generate measurements based on the imperial system.

In another example, flags may be used to identify how data is to be encrypted for transmission from application 106 to other external systems, such as decision support engine 112 or user database 110 illustrated in FIGs. 1A and 1B. For example, one value of an encryption flag may indicate that data is to be transmitted in the clear, a second value of the encryption flag may indicate that data is to be transmitted using a first form of encryption (e.g., a first set of keys associated with a first geographical region, a first encryption scheme, etc.), a third value of the encryption flag may indicate that data is to be transmitted using a second form of encryption (e.g., a second set of keys associated with a second geographical region, a second encryption scheme, etc.), and so on. In still another example, flags or other information may be set in the configuration information to control a periodicity at which the application 106 invokes various functions exposed by remote resources. Different values of the flags, or the setting of different flags, may indicate different periodicities at which data is to be reported or functions exposed by remote resources are to be invoked by application 106.

In yet another example, flags may be used to identify a setting of an on-boarding feature configured to provide a set of on-boarding instructions. For example, an on-boarding feature may have various sensor placement settings for providing different sensor placement instructions to users. Which setting is selected by application configurer 220 and indicated by a flag in the configuration information may be based, for example, on the user's location or other types of feature customization information. For example, where the feature customization information indicates that that user is in location X, application configurer 222 may generate configuration information including a flag that indicates a first setting for the application corresponding to location X. The first setting may then configure application 106 to provide instructions to the user to wear the sensor on their arms. In another example, if the user is in location Y, a second setting corresponding to location Y may configure application 106 to provide instructions to the user to wear the sensor either on their arms or their abdomen.

In another example, flags may be used to identify a setting of an application logging feature for logging application events. Examples of application events include application exceptions, major events (e.g., startups, stops, restarts, and security events), error event notifications, debugging information, SQL logs, warnings about low disk space, etc. A logging feature may have various logging settings corresponding to various levels of logging. For example, a first setting may correspond to a high logging level and a second setting may correspond to a low logging level. Which setting is selected by application configurer 222 for a certain user may depend on the feature customization information associated with the user, which may indicate the location of the user, application version of application 106, operating system of the user's mobile device, etc. As an example, if users in location X report a lot of technical issues, when new users in location X download application 106, application configurer 222 may configure the application for those users with the first setting so that a larger amount of application events can be obtained and analyzed by technical teams in charge of maintaining the application. A second setting corresponding to a low logging level, however, may be used for users in locations where a lower number of technical issues is reported.

Flags can be used to indicate the enablement or disablement of many different features and feature settings. In addition to the example features and feature settings provided above, some other example features include an alert feature, a BlueTooth communication feature, a security feature, a time provider feature, a secure networking application programming interface (API), a bulk data feature (associated with how data is transmitted from the sensor system 104 to application 106 in bulk), etc.

The configuration information may be generated as a structured file parseable by application 106. For example, the configuration information may be generated as an eXtensible Markup Language (XML) file, a JavaScript Object Notation (JSON) message, a graph data structure, or other parseable data that application 106 can use to identify which assets are to be activated in application 106 (e.g., which features to activate within application 106 and which resources with which the application 106 is to communicate). In some aspects, the configuration information may be encrypted prior to transmission to application 106 using a cryptographic key associated with the GCS 140 and/or the user of the application 106 to protect the cryptographic information from modification during transmission from application configurer 222 to application 106.

FIG. 4 is a message flow diagram 400 illustrating an example of messages exchanged between an application (such as application 106 illustrated in FIGs. 1A and 2), an authentication service (such as authentication service 130 illustrated in FIGs. 1A and 2), and a GCS (such as GCS 140 illustrated in FIGs. 1A and 2) to configure the application through the GCS, according to some embodiments described herein.

As illustrated, the application 106 may begin by loading 402 a login widget in which a user provides user credentials to be authenticated by the authentication service 130. After the user provides the user credentials 404 and submits the credentials in a login request 406 through the login widget, the authentication service 130 validates 408 the user credentials included in the login request 406. If the user credentials correspond to an existing user account, the authentication service 130 can generate and issue an access token 410 (or access information in a form other than a token) that the application 106 can use to request configuration of the application through the GCS 140. Otherwise, the authentication service 130 can generate an error message (not shown) which the application 106 can use to indicate that the user credentials were invalid and request entry of another set of user credentials.

After receiving the access token from the authentication service 130, the application 106 can request configuration information from the GCS 140 by transmitting, to the GCS 140, a request for URLs 412 (or an identification of remote resources with which the application 106 is to be provisioned). Generally, the request for URLs 412 includes the access token 410 provided by the authentication service 130 and provided to application 106. In response, the GCS 140 validates the token and, upon determining that the token is valid, transmits a URL configuration information response 414 including the URLs (or other identification of remote resources) with which the diabetes intervention application is allowed to communicate, based at least on location information included in the access token. Similarly, the application 106 can request configuration information from the GCS 140 by transmitting, to the GCS 140, an asset request 416 for information identifying application assets that are enabled for the user. This request also includes the access token provided by the authentication service. In response, the GCS 140 validates the token and, upon determining that the token is valid, transmits an asset configuration response 418 including various flags corresponding to assets that are activated or disabled for the user. The values of the flags corresponding to assets that are activated for the user may be set based on mapping information (e.g., configuration rules) identifying assets that are enabled for the location of the user and/or mapping information identifying optional assets that the user has enabled. Note that, in certain embodiments, a set of features and feature settings (associated with the application that is downloaded by the user) correspond to core features and feature settings. In certain embodiments, these core features and feature settings may be enabled for all users regardless of the user specifications and what the feature customization information indicates. As such, in such embodiments, the configuration information may not include any flags corresponding to the core features and feature settings.

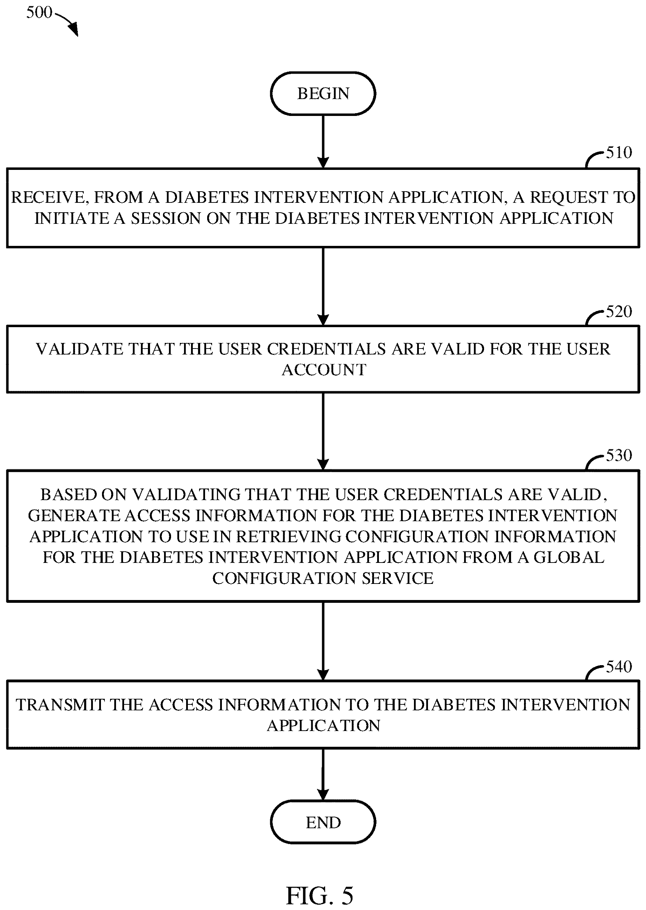

FIG. 5 illustrates example operations 500 that may be performed by an authentication service (such as authentication service 130 illustrated in FIGs. 1A and 2) to authenticate a user of a diabetes intervention application (such as application 106 illustrated in FIGs. 1A and 2) configured through a GCS (such as GCS 140 illustrated in FIGs. 1A and 2), according to some embodiments disclosed herein.

As illustrated, operations 500 begin at block 510, where the authentication service receives, from the diabetes intervention application, a request to initiate a session on the diabetes intervention application. The request generally includes user credentials for a user account associated with a user of the diabetes intervention application.

At block 520, the authentication service validates that the user credentials are valid for the user account. Validating the user credentials generally involves at least determining whether the provided username and password match credentials in a record in the user database. In some aspects, multifactor authentication may be used in which additional information, such as a user biometric or a one-time authentication code, is also validated in order to validate that the user credentials are valid for the user account.

At block 530, the authentication service generates access information for the diabetes intervention application to use in retrieving configuration information for the diabetes intervention application from a GCS. The access information is generated based on validating that the user credentials are valid for the user account.

At block 540, the authentication service transmits the access information to the diabetes intervention application. The access information may be transmitted, in some aspects, over an encrypted channel so that the access information is protected against modification during transit between the authentication service and the mobile device on which the diabetes intervention application executes.

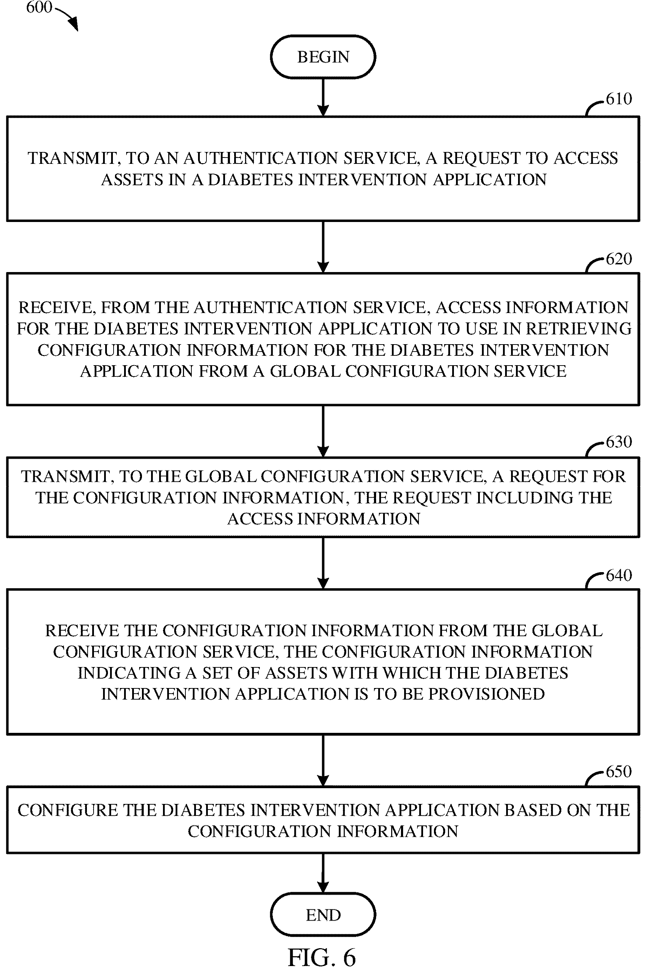

FIG. 6 illustrates example operations 600 that may be performed by a diabetes intervention application 106 illustrated in FIGs. 1A and 2 to configure the application through GCS 140 illustrated in FIGs. 1A and 2, according to some embodiments described herein.

As illustrated, operations 600 begin at block 610, where the application transmits, to an authentication service, a request to access assets associated with or in a diabetes intervention application. The request generally includes user credentials for a user of the diabetes intervention application. The user credentials generally include a username and password or access code, and may optionally include other information used in multi-factor authentication, such as a biometric or a one-time code provided by the authentication service.

At block 620, the application receives, from the authentication service, access information. The access information may be used by the diabetes intervention application to request configuration information from the GCS and may include information associated with the user. In some aspects, the access information may be encrypted using a cryptographic key known to the authentication service and the GCS, and the application can pass the access information to the GCS without decrypting the access information.

At block 630, the application transmits, to the GCS, a request for the configuration information. The request generally includes the access information received from the authentication service, which the GCS uses to determine whether the request is a legitimate request (as discussed above).

At block 640, the application receives the configuration information from the GCS. The configuration information generally includes information identifying a set of assets with which the diabetes intervention application is to be provisioned. As discussed, in one illustrative and non-limiting example, the set of assets may be determined based, at least in part, on location information included in the access token. Thus, the diabetes intervention application may be provisioned with assets that are appropriate for the geographic location associated with the user of the application and may not be provisioned with assets that are not supported in the geographic location associated with the user of the application.

At block 650, the application is configured based on the configuration application. For example, the diabetes intervention application can parse the configuration information to identify which features and feature settings to activate or deactivate and to identify the remote resources (e.g., URLs) with which the diabetes intervention application is allowed to communicate. Once configured, user interface features, aspects, pages, etc., associated with the activated features and the identified remote resources may be provided and displayed by the diabetes intervention application to the user (e.g., in response to user input, automatically, or through other mechanisms).

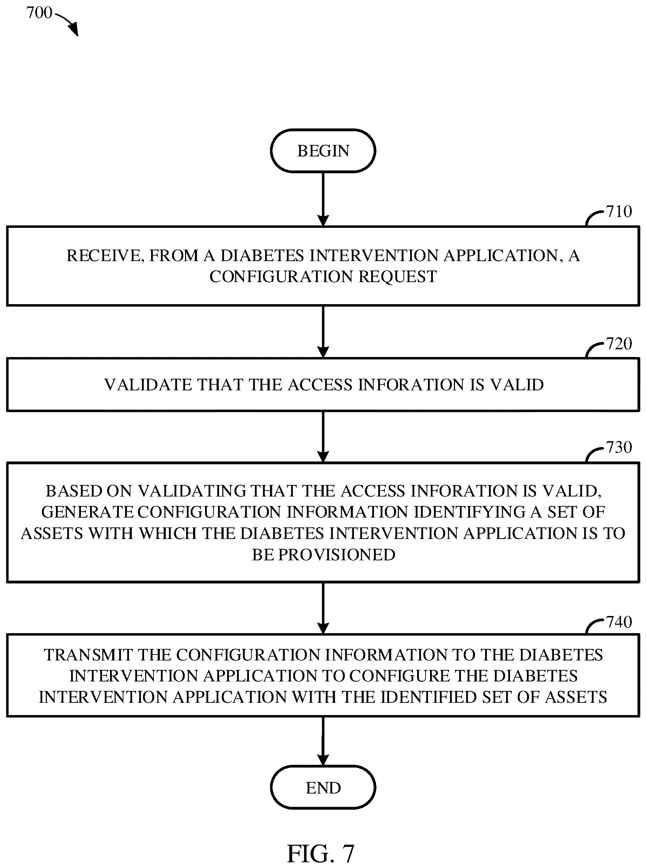

FIG. 7 illustrates example operations 700 that may be performed by GCS 140 illustrated in FIGs. 1A and 2, to configure a diabetes intervention application, according to some embodiments described herein.

As illustrated, operations 700 may begin at block 710, where the GCS receives, from a diabetes intervention application, a configuration request. The configuration request may, in some aspects, be a request for configuration information that can be used by the diabetes intervention application for enabling or disabling certain features, feature settings, and/or resources.

At block 720, the GCS validates that the access information included in the configuration request is valid. As discussed, valid access information may be access information that the GCS can decrypt into known or intelligible data or access information for which a matching authentication code can be generated.

At block 730, the GCS generates configuration information identifying a set of assets with which the diabetes intervention application is to be provisioned based on validating that the access information is valid. The configuration information may be generated based, at least in part, on the feature customization information included in the access information. For example, the configuration information may be generated based on location information included in the feature customization information, application version information, information about the environment in which the diabetes intervention application operates, device hardware or firmware information, sensor hardware or firmware information, user disease information, and other information that may be relevant to configuring the diabetes intervention application. In certain embodiments, the configuration information includes flags identifying features of the diabetes intervention application that are enabled and/or pointers (or links) to remote resources that the diabetes intervention application is to use during execution.

At block 740, the GCS transmits the configuration information to the diabetes intervention application to configure the diabetes intervention application with the set of assets indicated by the configuration information. As discussed, the diabetes intervention application is generally configured by the GCS transmitting the configuration information to the diabetes intervention application. The diabetes intervention application can parse the configuration information included in the parseable file to identify which features and feature settings to activate or deactivate and to identify the remote resources (e.g., URLs) with which the diabetes intervention application is allowed to communicate.

Note that in certain embodiments described above, an application, such as the diabetes intervention application, is downloaded on a user device (e.g., mobile device 107) and is later configured using the configuration information generated by the GCS. However, in certain other embodiments, when the user accesses the application store, a single, unified binary of the diabetes intervention application is not downloaded at that time on to the user device. In such embodiments, feature customization information is transmitted to the GCS, causing the GCS to map the feature customization information to a set of assets for the user. In such embodiments, instead of generating and transmitting configuration information to the application, the GCS transmits (directly or indirectly) the mapped set of assets to the user device, which downloads and executes the set of assets (corresponding to an application that is customized based on the user specifications).

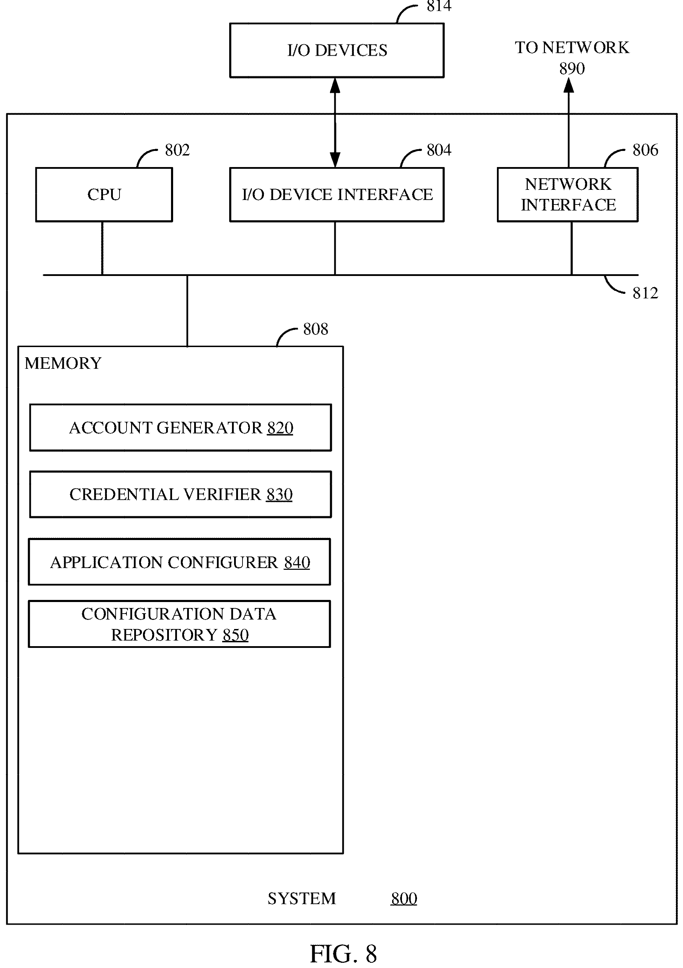

FIG. 8 illustrates an example system 800 for authenticating and configuring an application. For example, system 800 may correspond to one or more of the authentication service 130 and/or GCS 140 illustrated in FIGs. 1 and 2.

As shown, system 800 includes a central processing unit (CPU) 802, one or more I/O device interfaces 804 that may allow for the connection of various I/O devices 814 (e.g., keyboards, displays, mouse devices, pen input, etc.) to the system 800, network interface 806 through which system 800 is connected to network 890 (which may be a local network, an intranet, the internet, or any other group of computing devices communicatively connected to each other), a memory 808, and an interconnect 812.

CPU 802 may retrieve and execute programming instructions stored in the memory 808. Similarly, the CPU 802 may retrieve and store application data residing in the memory 808. The interconnect 812 transmits programming instructions and application data, among the CPU 802, I/O device interface 804, network interface 806, and memory 808.

CPU 802 is included to be representative of a single CPU, multiple CPUs, a single CPU having multiple processing cores, and the like.

Memory 808 is representative of a volatile memory, such as a random access memory, and/or a non-volatile memory, such as nonvolatile random access memory, phase change random access memory, or the like. As shown, memory 808 includes an account generator 820, credential verifier 830, application configurer 840, and configuration data repository 850. Account generator 820 generally receives requests to create user accounts for a diabetes intervention application and creates records in a user repository including, for each user of the application, at least user credentials and user location information that can be used to determine a configuration of the diabetes intervention application for the user. In some aspects, account generator 820 can additionally include, in a user record, other information that can be used to determine the configuration of the diabetes intervention application, including application version information, operating environment information, and other information relevant to a configuration of the application.

Credential verifier 830 generally receives login requests from an application executing on a mobile device and verifies that the credentials included in the received login requests match the credentials included in records generated by account generator 820. If the user credentials included in a received login request are determined to be valid, credential verifier generates an access token including information that application configurer 840 can use to configure the application. Credential verifier 830 then provides the generated access token to the application executing on the mobile device.

Application configurer 840 receives configuration requests from the application and uses the access token included in a configuration request and information stored in configuration data repository 850 to configure the application. Generally, to configure the application, application configurer 840 can search for configuration data from configuration data repository 850 based at least on location information included in the access token and generate a parseable file based on the retrieved configuration data. The configuration data generally includes information identifying remote resources with which the application can communicate and flags identifying whether assets in the application are enabled or disabled. The parseable file is then transmitted to the application for further processing, which results in the application being configured with assets that are appropriate for the location associated with the user of the application.

Accordingly, certain embodiments described herein provide a technical solution to a technical problem in the technical field of personalizing software application configuration based on user information (e.g., feature customization information). For example, certain embodiments relate to deploying a single, unified collection of available assets (e.g., features, feature settings, and/or resources) for an application, regardless of user-specific specifications. Storing, maintaining, and provisioning a single, unified application to different users, regardless of user-specific specifications leads to significant resource efficiency. More specifically, a large amount of storage resources may be freed and used for other purposes if a single application binary and codebase are stored and maintained, as opposed to many different ones with overlapping code. In addition, significantly less computing resources may be spent when updating, compiling, and debugging a single application binary and a single codebase, as opposed to many application binaries and codebases. Further, maintaining a single codebase reduces the likelihood of introducing errors into the code during code updates or resolving errors in some, but not all, versions of the application.

Further, as discussed above, some applications may be safety critical applications for which regulatory approval is needed before features are made available to users in a given location (e.g., to ensure that the new features do not harm patients using the software application, that the new features comply with security requirements or best practices, etc.). New features in a safety critical application may be approved in some regions earlier than in other regions. Thus, in deploying a new feature in a safety critical application, a developer can choose to deploy different versions of an application, with versions including the new feature being deployed in regions where regulatory approval has been obtained and versions excluding the new feature being deployed in regions where regulatory approval has not been obtained. When multiple codebases exist for multiple versions of an application, additional complexity may be introduced by adding new features in a piecemeal fashion (e.g., as features are approved by the relevant regulatory authorities).

Further, utilizing the systems, methods, and techniques described herein allow for customizing a diabetes intervention application for a user based on the user's specifications. An application with functionalities, features, feature settings, and resources that are customized to a user's unique preferences and needs will provide a more personalized experience and be much more useful, beneficial, and engaging to a user, thereby helping improve the user's health.

Each of these non-limiting examples can stand on its own or can be combined in various permutations or combinations with one or more of the other examples. The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Geometric terms, such as "parallel", "perpendicular", "round", or "square", are not intended to require absolute mathematical precision, unless the context indicates otherwise. Instead, such geometric terms allow for variations due to manufacturing or equivalent functions. For example, if an element is described as "round" or "generally round", a component that is not precisely circular (e.g., one that is slightly oblong or is a many-sided polygon) is still encompassed by this description.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. § 1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

The description can be further exemplified by the following clauses:
1. A method for configuring a health intervention application executing on a computing device via a remote server, comprising:
   receiving, from the health intervention application, a request for assets provided by the remote server, the request including access information associated with a user of the computing device, the access information including feature customization information for the health intervention application and being issued by an account management service for the health intervention application;
   validating that the access information is valid;
   responsive to validating that the access information is valid, generating configuration information identifying a set of assets with which the health intervention application is to be provisioned based, at least in part, on the feature customization information included in the access information; and
   transmitting the configuration information to the health intervention application to configure the health intervention application with the identified set of assets.
2. The method of clause 1, wherein the feature customization information comprises location information associated with a geographical location of the user of the computing device.
3. The method of clause 1, wherein the generating comprises:
   based on the feature customization information, identifying, from a plurality of uniform resource locators (URLs), a set of URLs to be used for configuring the health intervention application, wherein the configuration information includes the set of URLs.
4. The method of clause 1, wherein the generating comprises:
   based on the feature customization information, identifying, from a plurality of application features, a set of application features to be activated for configuring the health intervention application, wherein the configuration information includes one or more flags associated with set of application features set to a value indicating that the set of application features are activated.
5. The method of clause 1, wherein the feature customization information includes version information for the health intervention application executing on the computing device.
6. The method of clause 1, wherein the feature customization information includes operating environment information for the computing device and glucose monitoring devices connected with the computing device.
7. The method of clause 1, wherein the access information compṙises an access token including the feature customization information and token authentication information.
8. The method of clause 1, wherein the configuration information comprises a mapping of application parameters associated with application features to values corresponding to specific application feature settings.
9. A computing system, comprising:
   at least one memory comprising executable instructions; and
   at least one processor in data communication with the at least one memory and configured to execute the instructions to cause the computing system to:
      receive, from a health intervention application executing on a computing device, a request for assets provided by the computing system, the request including access information associated with a user of the computing device, the access information including feature customization information for the health intervention application and being issued by an account management service for the health intervention application;
      validate that the access information is valid;
      responsive to validating that the access information is valid, generate configuration information identifying a set of assets with which the health intervention application is to be provisioned based, at least in part, on the feature customization information included in the access information; and
      transmit the configuration information to the health intervention application to configure the health intervention application with the identified set of assets.
10. The computing system of clause 9, wherein the feature customization information comprises location information associated with a geographical location of the user of the computing device.
11. The computing system of clause 9, wherein the at least one processor being configured to cause the computing system to generate the configuration information comprises the at least one processor being configured to cause the computing system to:
   based on the feature customization information, identify, from a plurality of uniform resource locators (URLs), a set of URLs to be used for configuring the health intervention application, wherein the configuration information includes the set of URLs.
12. The computing system of clause 9, wherein the at least one processor being configured to cause the computing system to generate the configuration information comprises the at least one processor being configured to cause the computing system to:
   based on the feature customization information, identify, from a plurality of application features, a set of application features to be activated for configuring the health intervention application, wherein the configuration information includes one or more flags associated with set of application features set to a value indicating that the set of application features are activated.
13. The computing system of clause 9, wherein the feature customization information includes version information for the health intervention application executing on the computing device.
14. The computing system of clause 9, wherein the feature customization information includes operating environment information for the computing device and glucose monitoring devices connected with the computing device.
15. The computing system of clause 9, wherein the access information comprises an access token including the feature customization information and token authentication information.
16. The computing system of clause 9, wherein the configuration information comprises a mapping of application parameters associated with application features to values corresponding to specific application feature settings.
17. A non-transitory computer readable medium having instructions stored thereon that, when executed by a computing system, cause the computing system to perform a method comprising:
   receiving, from a health intervention application executing on a computing device, a request for assets provided by the computing system, the request including access information associated with a user of the computing device, the access information including feature customization information for the health intervention application and being issued by an account management service for the health intervention application;
   validating that the access information is valid;
   responsive to validating that the access information is valid, generating configuration information identifying a set of assets with which the health intervention application is to be provisioned based, at least in part, on the feature customization information included in the access information; and
   transmitting the configuration information to the health intervention application to configure the health intervention application with the identified set of assets.
18. The non-transitory computer readable medium of clause 17, wherein the feature customization information comprises location information associated with a geographical location of the user of the computing device.
19. The non-transitory computer readable medium of clause 17, wherein the generating comprises:
   based on the feature customization information, identifying, from a plurality of uniform resource locators (URLs), a set of URLs to be used for configuring the health intervention application, wherein the configuration information includes the set of URLs.
20. The non-transitory computer readable medium of clause 17, wherein the generating comprises:
   based on the feature customization information, identifying, from a plurality of application features, a set of application features to be activated for configuring the health intervention application, wherein the configuration information includes one or more flags associated with set of application features set to a value indicating that the set of application features are activated.

## Claims

1. A method for configuring a health intervention application executing on a computing device, comprising:
transmitting, to an authentication service, a request to access assets in the health intervention application, wherein the assets comprise features, feature settings, and/or resources of the health intervention application;
receiving, from the authentication service, access information for the health intervention application to use in retrieving configuration information for the health intervention application from a configuration service, wherein the access information is associated with a user of the computing device and includes feature customization information for the health intervention application, and wherein the feature customization information comprises user specifications;
transmitting, to the configuration service, a request for the configuration information, the request including the access information;
receiving the configuration information from the configuration service, the configuration information indicating a set of assets with which the health intervention application is to be provisioned; and
configuring the health intervention application based on the configuration information.

2. The method of claim 1, wherein the feature customization information comprises location information associated with a geographical location of the user of the computing device.

3. The method of claim 1 or claim 2, wherein the configuration information includes one or more uniform resource locators (URLs) to be used for configuring the health intervention application.

4. The method of any preceding claim, wherein the configuration information includes one or more flags associated with a set of application features of a plurality of application features to be activated for configuring the health intervention application.

5. The method of any preceding claim, wherein the feature customization information includes version information for the health intervention application executing on the computing device.

6. The method of any preceding claim, wherein the feature customization information includes operating environment information for the computing device and a glucose monitoring device connected with the computing device.

7. The method of any preceding claim, wherein the access information comprises an access token including the feature customization information and token authentication information.

8. The method of any preceding claim, wherein the configuration information comprises a mapping of application parameters associated with application features to values corresponding to specific application feature settings.

9. A computing system, comprising:
at least one memory comprising executable instructions; and
at least one processor in data communication with the at least one memory and configured to execute the instructions to cause the computing system to:
transmit, to an authentication service, a request to access assets in the health intervention application, wherein the assets comprise features, feature settings, and/or resources of the health intervention application;
receive, from the authentication service, access information for the health intervention application to use in retrieving configuration information for the health intervention application from a configuration service, wherein the access information is associated with a user of the computing device and includes feature customization information for the health intervention application, and wherein the feature customization information comprises user specifications;
transmit, to the configuration service, a request for the configuration information, the request including the access information;
receive the configuration information from the configuration service, the configuration information indicating a set of assets with which the health intervention application is to be provisioned; and
configure the health intervention application based on the configuration information.

10. The computing system of claim 9, wherein the feature customization information comprises location information associated with a geographical location of the user of the computing device.

11. The computing system of claim 9 or claim 10, wherein the configuration information includes one or more uniform resource locators (URLs) to be used for configuring the health intervention application.

12. The computing system of any of claims 9 to 11, wherein the configuration information includes one or more flags associated with a set of application features of a plurality of application features to be activated for configuring the health intervention application.

13. The computing system of any of claims 9 to 12, wherein the feature customization information includes version information for the health intervention application executing on the computing device.

14. The computing system of any of claims 9 to 13, wherein the feature customization information includes operating environment information for the computing device and a glucose monitoring device connected with the computing device.

15. A non-transitory computer readable medium having instructions stored thereon that, when executed by a computing system, cause the computing system to perform a method of any of claims 1 to 8.
